# EUROPEAN PATENT SPECIFICATION

(11) **EP 1 992 689 B1**
(45) Date of publication and mention of the grant of the patent: **18.03.2015**
(21) Application number: 07714626.4
(22) Date of filing: 14.02.2007
(51) Int. Cl.: C12N 15/00, C12N 15/09

(54) **METHOD FOR EXTRACTION OF NUCLEIC ACID FROM BIOLOGICAL MATERIAL**
VERFAHREN ZUR EXTRAKTION VON NUKLEINSÄURE AUS BIOLOGISCHEM MATERIAL
PROCÉDÉ D'EXTRACTION D'ACIDE NUCLÉIQUE À PARTIR D'UN MATÉRIAU BIOLOGIQUE

(30) Priority: 15.02.2006 JP 2006038276
(43) Date of publication of application: 19.11.2008
(73) Proprietor: Tosoh Corporation, Yamaguchi 746-8501 (JP)
(72) Inventor: TSUCHIYA, Shigeo, Machida-shi, Tokyo 1940003 (JP); HAYASHI, Toshinori, Sagamihara-shi, Kanagawa 2280804 (JP)
(74) Representative: Pautex Schneider, Nicole Véronique
(86) International application number: PCT/JP2007/053126
(87) International publication number: WO 2007/094506

(56) References cited:
- WO-A-01/34844
- WO-A-01/45522
- WO-A-95/01359
- WO-A-99/61603
- JP-A- 05 068 553
- JP-A- 09 505 479
- JP-A- 10 191 978
- US-A1- 2006 234 251
- TSUCHIYA S. ET AL.: 'Kosankin Kakusan Chushutsu Shiyaku EXTRAGEN MB no Kaihatsu' TOSOH RESEARCH & TECHNOLOGY REVIEW vol. 50, December 2006, pages 63 - 68, XP003016664

### SEQUENCE LISTING

<110> TOSOH CORPORATION
<120> Method for Extraction of Nucleic Acid from Biological M aterial
<130> TYS-S935
<150> JP2006-038276
   <151> 2006-02-15
<160> 11
<210> 1
   <211> 24
   <212> DNA
   <213> Artificial Sequence
<220>
   <223> MYR-1S-10
<400> 1
   tttccgttcg acttgcatgt gtta 24
<210> 2
   <211> 51
   <212> DNA
   <213> Artificial Sequence
<220>
   <223> MYR-1F-10
<400> 2
   aattctaata cgactcacta tagggagacg gaaaggtctc ttcggagata c 51
<210> 3
   <211> 18
   <212> DNA
   <213> Artificial Sequence
<220>
   <223> MYR-3RT18
<400> 3
   acaagacatg catcccgt 18
<210> 4
   <211> 18
   <212> DNA
   <213> Artificial Sequence
<220>
   <223> YO-MYR-P2-2-S-G
<400> 4
   cgaagtgcag ggcagatc 18
<210> 5
   <211> 24
   <212> DNA
   <213> Artificial Sequence
<220>
   <223> MYR-1S-40
<400> 5
   ccgccagcgt tcgtcctgag ccag 24
<210> 6
   <211> 51
   <212> DNA
   <213> Artificial Sequence
<220>
   <223> MYR-1F-40
<400> 6
   aattctaata cgactcacta tagggagatg gcggcgtgct taacacatgc a 51
<210> 7
   <211> 16
   <212> DNA
   <213> Artificial Sequence
<220>
   <223> MYR-3RA16-4
<400> 7
   gacatgcgtc ttgagg 16
<210> 8
   <211> 18
   <212> DNA
   <213> Artificial Sequence
<220>
   <223> YO-MYR-P5-S-G
<400> 8
   caaattgccc acgtgtta 18
<210> 9
   <211> 18
   <212> DNA
   <213> Artificial Sequence
<220>
   <223> MYR-3RI18
<400> 9
   taaagacatg cgcctaaa 18
<210> 10
   <211> 16
   <212> DNA
   <213> Artificial Sequence
<220>
   <223> MYR-3RK16-4
<400> 10
   ggcatgcgcc aagtgg 16
<210> 11
   <211> 15
   <212> DNA
   <213> Artificial Sequence
<220>
   <223> YO-MYR-P4-S-G
<400> 11
   ccggtgtgca gggca 15

## Description

### FIELD OF THE INVENTION

The present invention relates to a method of extracting nucleic acid to be subjected to a nucleic acid amplification reaction from biological material in a biological sample in a simple and quick method, and can be applied to medical fields such as infection testing, research fields such as molecular biology, industrial fields such as food safety management, and the like. Specifically the present invention permits the simple and efficient extraction of nucleic acid from biological materials such as *Mycobacterium* having a rigid cell wall, and subsequent genetic diagnosis using a nucleic acid amplification reaction.

### BACKGROUND OF THE INVENTION

Conventionally, when nucleic acid is prepared from a sample of interest, it is commonly performed in two steps of extraction and purification, wherein suitable extraction and purification steps are selected depending on the properties of the sample.

As general methods of extracting nucleic acid from biological materials such as viruses, microorganisms, protozoa, plant and animal tissues, there are mechanical methods using a homogenizer etc., chemical methods using a denaturing agent etc., and biological methods using an enzyme etc. There are also methods in which a particulate solid is mixed with a frozen sample, and the sample is agitated to disrupt the cells. Among them, extraction methods using denaturing agents such as guanidine hydrochloride and guanidine thiocyanate (see Molecular Cloning: A Laboratory Manual, Appendix 7.23-7.25, New York Laboratory, 1989) contain a denaturing agent in the extract, and the agent per se inhibits the amplification reaction, and thus a purification step after extraction is required. Nucleic acid purification by enzyme treatment is time-consuming, and specifically when it is applied to materials having rigid cell walls such as bacteria of the genus *Mycobacterium* and spores of molds such as fungi, cells cannot be easily disrupted and extraction efficiency of nucleic acid is extremely poor.

Thus, in order to overcome the problem of a cell wall that is rigid and thus cell disruption is difficult, a method of adding microparticles and conducting ultrasonication using an ultrasonic bath to release nucleic acid has been disclosed (see U.S. Pat. No. 5,374,522). This releasing method is simple and has a good disruption efficiency. However, when a test sample such as sputum or feces is used to extract nucleic acid, and the extracted nucleic acid is used for gene amplification, a further purification process is required to remove inhibitors to the amplification reaction.

Thus, in order to perform the rapid processing of a large number of test samples, a nucleic acid extraction method has been sought, which permits a simple and efficient separation of nucleic acid from biological materials and renders the extracts available for gene amplification reaction.

### DISCLOSURE OF THE INVENTION

As test samples to be used in gene testing, there can be mentioned blood, urine, sputum, pus, blood culture, swab, colonies and the like. Nucleic acid extracted from these test samples may be subjected to direct testing. However, in recent years in nucleic acid testing, it is more common to amplify nucleic acid extracted from test samples using various amplification reactions prior to measurement in order to enable the qualification and quantitation of minute amounts of nucleic acid. Examples of such nucleic acid amplification reactions include RNA amplification methods such as the Nucleic Acid Sequence Based Amplification (NASBA) method as described in Japanese Patent No. 2650159, the so-called Transcription-Mediated Amplification (TMA) method as described in Japanese Unexamined Patent Publication (Kohyo) No. 4-500759, and the Transcription-Reverse transcription Concerted reaction (TRC) as described in Japanese Unexamined Patent Publication (Kokai) No. 2000-14400 (Patent application No. 10-186434) which is the method of Examples of the present invention, and the DNA amplification methods such as the polymerase chain reaction (PCR).

It is also possible to extract nucleic acid from the various test samples described above by conventional nucleic acid-extraction methods as described above. However, as the frequency of subjecting nucleic acid extracts to amplification reactions as described above increases, there are more chances that nucleic acid extracts obtained by a conventional nucleic acid extraction method, contain nucleic acid amplification reaction-inhibiting substances that inhibit the activity or reaction of enzymes, such as reverse transcriptase and DNA polymerase for use in nucleic acid amplification reactions as described above. It has been pointed out that sputum, among the above-mentioned test samples is more likely to contain nucleic acid amplification reaction-inhibiting substances derived from biological components such as airway mucus, various nucleic acids and cells, and when they contaminate the nucleic acid extract, nucleic acid amplification reaction is inhibited, resulting in accurate testing.

In gene testing, internal standards are usually used and false negatives can be prevented. However, it is clear that the most desirable solution is to remove nucleic acid amplification reaction-inhibiting substances during the nucleic acid extraction process.

Thus, it is an object of the present invention to provide a method of extracting nucleic acid, simply and efficiently, from bacteria having a rigid cell wall, such as *Mycobacterium,* which is carried out after removing substances that inhibit nucleic acid amplification reactions from biological materials from which the nucleic acid is extracted, and a kit for conducting the method.

The first invention of the present application to achieve the above objective is a method of extracting biological material-derived nucleic acid from samples containing biological material, the method comprising mixing biological material with an aqueous solution containing at least a surfactant, subjecting the mixture to a heating treatment and then removing the aqueous solution to separate the biological material, adding a solid powder suspension to the biological material, agitating or sonicating it to disrupt the biological material, and extracting nucleic acid from the biological material. The second invention of the present application is as claimed in the above first invention, wherein the surfactant is an anionic surfactant having a steroid backbone or an ampholytic surfactant. The third invention of the present application is as claimed in the above second invention, wherein the anion surfactant or ampholytic surfactant is selected from the group consisting of bile acid, cholic acid, deoxycholic acid, 3-[(3-cholamidopropyl)dimethylammonio]propane sulfonic acid (CHAPS) and salts thereof. In a fourth invention of the present application, the concentration of the surfactant is in the range of 0.1 mM to 50 mM. In a fifth invention of the present application, the temperature of the heating treatment is 60°C to 90°C. In a sixth invention of the present application, the above solid powder is amorphous, its long diameter is 32 µm or less, its specific gravity is 3.5 or greater, and its hardness (Hv10) is 600 or greater. In a seventh invention of the present application, the above amorphous powder is zirconia. An eighth invention of the present application is a method of extracting biological material-derived nucleic acid from samples containing the biological material, the method comprising the steps of: (1) separating the biological material from the above sample, (2) mixing the biological material with an aqueous solution containing, at least a cholic acid at a concentration of 0.1 to 50 mM, (3) subjecting the mixture to 60°C to 90°C and then removing the aqueous solution to separate the above biological material, (4) adding to the biological material a suspension containing zirconia powder, wherein the powder has an amorphous shape and has a major diameter of 20 µm or less, a specific gravity of 4.5 to 6.5, and a hardness (Hv10) of 800 or greater, (5) disrupting the above biological material by ultrasonication, and (6) obtaining the nucleic acid of interest in the supernatant of the disrupted product. In a ninth invention of the present application, the above ultrasonication is conducted by treating simultaneously or alternately with ultrasonic waves of two wavelengths. In a tenth invention of the present application, the above biological material is a virus, a microorganism, a protozoa, a plant or an animal tissue. In an eleventh invention of the present application, the above microorganism is one that belongs to the genus *Mycobacterium.* In a twelfth invention of the present application, the sample containing the biological material is an organism-derived from a sample, such as sputum, gastric juice, urine, pus, ascites, pleural effusion, pericardial fluid, blood and tissue, tissue washing liquid such as a bronchial lavage and an alveolar lavage, culture medium, and an environmental material such as soil, water and air. A thirteenth invention of the present application is a reagent kit for conducting the method of nucleic acid extraction as claimed in the above inventions 1-12, wherein the kit comprises at least a reagent containing the above surfactant and a reagent containing the above solid powder as a constituent. The present invention will now be explained in detail below.

### BRIEF EXPLANATION OF THE DRAWINGS

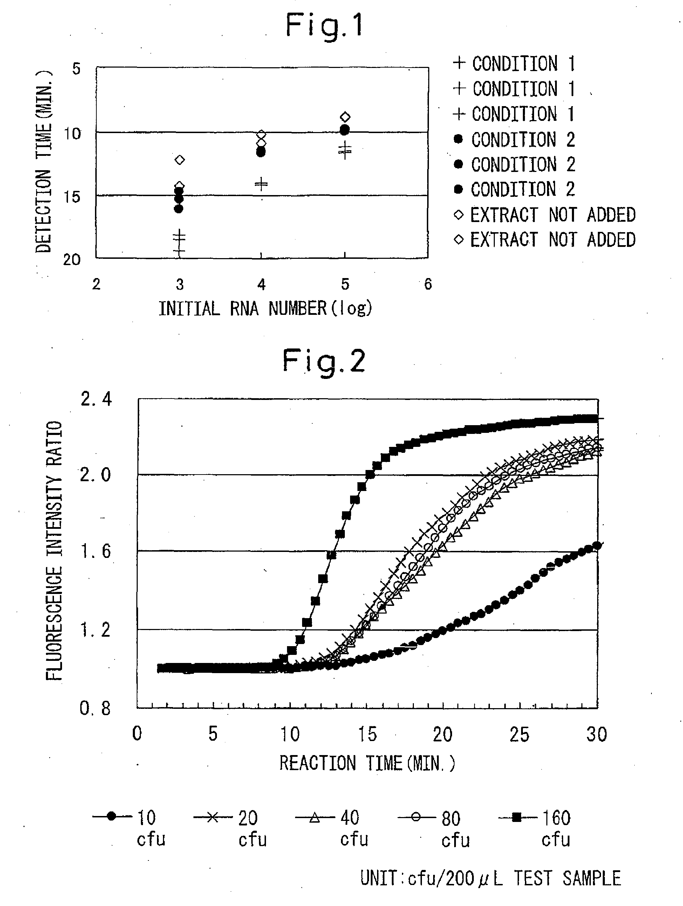

Fig. 1 shows the result of a TRC reaction conducted in the method of Example 1, in which various concentrations of standard RNA of *Mycobacterium tuberculosis* 16S rRNA were added to a nucleic acid extract from a NALC-treated sputum and then subjected to the TRC reaction.
Fig. 2 shows a graph showing the reaction time and the fluorescence intensity ratio of an RNA amplification reaction conducted in Example 2, wherein test samples in which 10 to 160 cfu (colony forming unit) BCG were dispersed in 200 µl of a tuberculosis-negative NALC-treated product, from which nucleic acid was extracted, and the extract obtained was subjected to the RNA amplification reaction. Test samples up to bacteria 10 cfu/200 µl were detected.

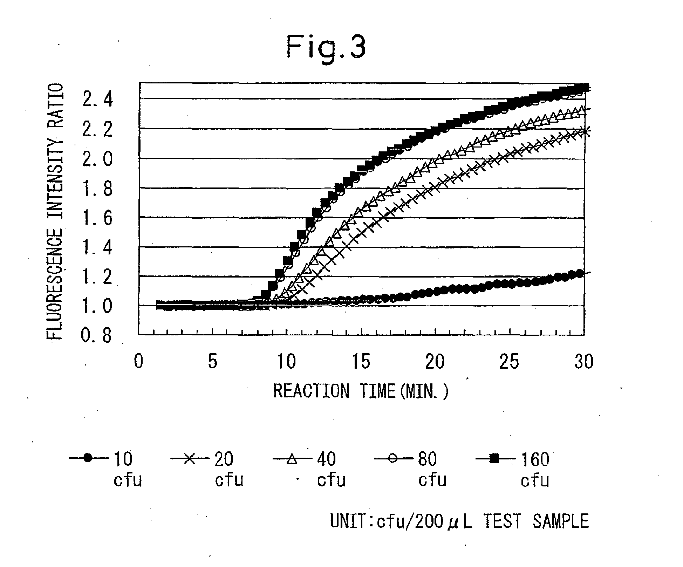

Fig. 3 shows a graph showing the reaction time and the fluorescence intensity ratio of a RNA amplification reaction conducted in Example 3 wherein test samples in which 10 to 160 cfu of *Mycobacterium avium* were dispersed in 200 µl of a tuberculosis-negative NALC-treated product, from which nucleic was acid extracted, and the extract obtained was subjected to the RNA amplification reaction. Test samples up to bacteria 10 cfu/200 µl were detected.

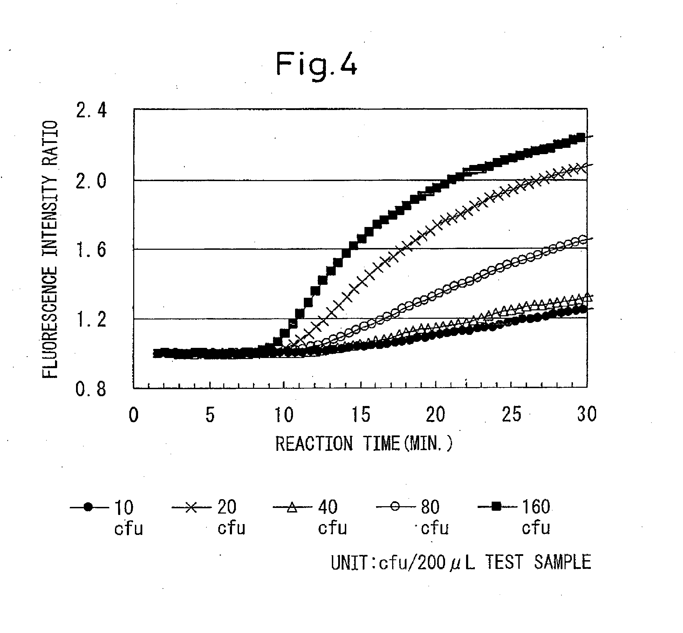

Fig. 4 shows a graph showing the reaction time and the fluorescence intensity ratio of an RNA amplification reaction conducted in Example 4 wherein test samples in which 10 to 160 cfu of *Mycobacterium intracellulare* were dispersed in 200 µl of a tuberculosis-negative NALC-treated product, from which nucleic acid was extracted, and the extract obtained was subjected to the RNA amplification reaction. Test samples up to bacteria 10 cfu/200 µl were detected.

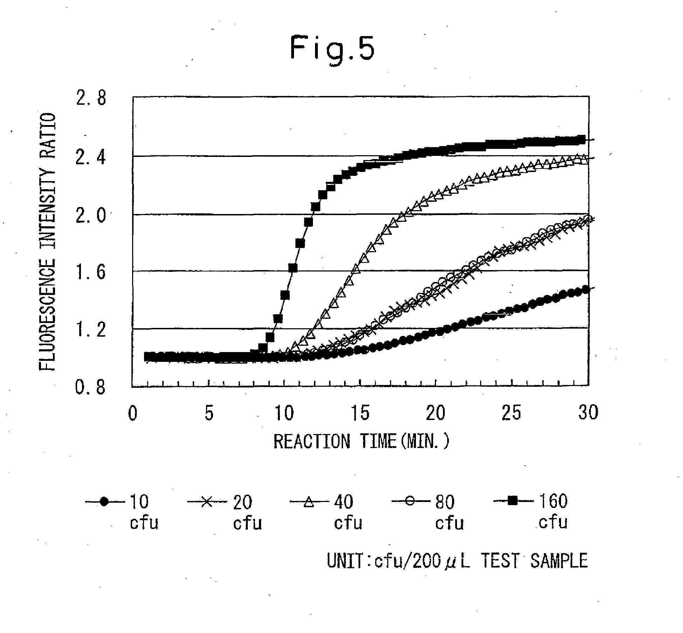

Fig. 5 shows a graph showing the reaction time and the fluorescence intensity ratio of an RNA amplification reaction conducted in Example 5 wherein test samples in which 10 to 160 cfu of *Mycobacterium kansasii* were dispersed in 200 µl of a sham sputum (mucin 2.1 mg/ml, peptone 4.2% solution), from which nucleic acid was extracted, and the extract obtained was subjected to the RNA amplification reaction. Test samples with up to 10 cfu/200 µl of bacteria were detected.

### BEST MODE FOR CARRYING OUT THE INVENTION

As test samples in the present invention, there can be mentioned sputum, gastric juice, urine, pus, alveolar lavage, ascites, pleural effusion, pericardial fluid, feces, tissue, blood, serum, colonies, swabs or other body fluids, and sample suspensions, such as homogenized food samples. When the sample is a sputum, a pretreatment such as a NALC treatment to lower the viscosity of the sample may be carried out to obtain a more preferred result. There can also be mentioned environmental water, waste water and soil in the environmental analysis.

As biological materials in the present invention, there can be mentioned, but not limited to, viruses, bacteria, fungi, protozoa, plants or animals. The present invention is useful for biological materials, such as fungi and *Mycobacterium* for which cell disruption is difficult.

While, in the nucleic acid extraction method of the present invention, substances that inhibit the nucleic acid amplification reaction are eluted with a surfactant and removed during the extraction of nucleic acid from test samples, the present inventors believe that one of the substances that inhibit the nucleic acid amplification reaction is glycoprotein, and thus by solubilizing glycoprotein with a surfactant, the effect of removing substances that inhibit the nucleic acid amplification reaction may be exhibited. As used herein, examples of such glycoproteins include airway mucus glycoprotein (called mucin) contained in sputum.

Examples of the surfactant for use in the present invention are not specifically limited as long as they are anionic surfactants having a steroid backbone or ampholytic surfactants, and preferably at least one surfactant selected from bile acid, cholic acid, deoxycholic acid, CHAPS and salts thereof. When cholic acid is used, concentrations of 0.1 mM to 50 mM, preferably 1 mM to 20 mM are used. The temperature of the heating treatment is preferably, but not limited to, 60°C to 90°C, more preferably 60°C to 80°C, and heating time is, but not limited to, preferably 2 minutes to 20 minutes, more preferably 2 minutes to 5 minutes. The heating treatment as used herein may preferably be conducted by being left at rest. The number of times of surfactant treatment preferably is, but not limited to, one to about five times. In order to mix nucleic acid-containing test samples with a surfactant and then selectively removing the eluent, a centrifugation process, for example, may be employed. The nucleic acid-containing test samples and the surfactant-eluent containing substances that inhibit nucleic acid amplification reactions can be separated by centrifuging at, but not limited to, 6,000xg for 5 minutes. In the process of the present invention of mixing a surfactant and then selectively removing the eluent, it is important to establish specific conditions taking into account the nucleic acid to be extracted and test samples subjected to extraction and conducting preliminary experiments.

The material of the solid powder of the present invention includes for example, but not limited to, zirconia, diamond, alumina (aluminum oxide), iron, an alloy and the like. However, from the viewpoint of extraction efficiency and cost, zirconia is preferred.

The shape of the above solid powder is not specifically limited as long as it can physically disrupt the biological material, but preferably is amorphous. Amorphous solid powder refers to a solid powder comprising particles that do not have smooth surfaces like a sphere or an ellipsoid, but have sharp convexo-concave surfaces. The size of the above particles should be at least 32 µm at the longest portion (long diameter) of the particle diameter, and preferably a mean of 20 µm or less (measuring instrument: COULTER LS130). Its specific gravity is preferably as large as 3.0 or greater, since it can disrupt tissues using the kinetic energy of the powder by ultrasound waves or agitation. The supernatant containing extracted nucleic acid and the above solid powder can be separated by centrifugation or being left at rest. By including magnetic bodies in the solid powders in advance, magnetic separation may be effected. The greater the hardness of the solid powder is, the better, and it is preferably 600 or greater in terms of Vickers' hardness (Hv10). In a preferred embodiment of the present invention, an amorphous solid powder of zirconia having a long diameter of 20 µm or less, a specific gravity of 5.7 to 6.2, and a hardness (Hv10) of 900 or greater is used. Examples of an amorphous powder of zirconia include, but not limited to, fused zirconia ZCO-E6 (manufactured by ASTRON, mean long diameter of 6 µm, specific gravity of 5.8, Vickers' hardness (Hv10) 1200), fused zirconia NST 8H, F350 (manufactured by SAINT-GOBAIN, mean long diameter of 24 µm, specific gravity of 6.0, Vickers' hardness (Hv10) 1200), fused zirconia #400 (manufactured by PACIFIC-RUNDUM Co., Ltd., mean long diameter of 18 µm) and the like. The amount of the solid powder added to the sample is determined as appropriate depending on the type of the sample, the method of agitation, and the time of agitation. After adding the solid powder, agitation and/or ultrasonication may be conducted to physically disrupt the cells.

The method of agitation may be manual reciprocating motion of a container having a sample therein, and the direction of the motion may be either vertical or horizontal, and preferably conducted in a vertical reciprocating motion. Alternatively, it may be agitated with a vortex mixer. For the disruption of cells, ultrasonic disruption is more preferred, and in order to eliminate the effect of a stationary wave, alternate or simultaneous ultrasonication with 2 frequencies or more is most preferred. For ultrasonic treatment, a container holding the sample and the powdery solid is immersed in or floated on the liquid in the washing vessel of the ultrasonic cleaner. The agitating time or ultrasonication time may be until the cell membrane of the microorganism or the tissue is disrupted, and may be 2 minutes or longer. The time may be longer than this, when the tissue has a tenacious and rigid cell wall such as plant tissue.

In accordance with the present invention, nucleic acid may be harvested from cells in a purified form to the extent that the subject organism may be identified and detected by a nucleic acid amplification reaction. The present invention is also useful for various test samples that contain as the subject organism microorganisms having a rigid cell wall and various nucleic acid-inhibiting substances. For example, nucleic acid may be efficiently extracted from *Mycobacterium,* as well as contained in sputum, and a sensitive and rapid gene testing may be conducted.

### EXAMPLES

The present invention will now be explained in detail with reference to Examples below. It should be noted, however, that the present invention is not limited to these examples.

### Example 1

### An inhibiting substance present in nucleic acid-extracted samples from sputum

Two hundred microliters of a test sample prepared by subjecting sputum (hereinafter referred to as "tuberculosis-negative sputum") obtained with consent from a patient infected with nontuberculous acid-fast bacteria to a NALC-NaOH treatment (trade name: BBL Mycoprep, Nippon Becton Dickinson Co., Ltd) were used in the following two treatments. Condition 1: The sample was added to 1 ml of PBS, agitated, and then centrifuged at 16,000 x g for 5 minutes to remove the supernatant. Condition 2: The sample was added to 1 ml of 50 mM glycine-NaOH buffer (hereinafter referred to as "washing reagent") adjusted to pH 9 containing 10 mM sodium cholate and 1 mM EDTA, allowed to stand on a dry heat block at 80°C for 2 minutes, and then centrifuged at 16,000 x g for 5 minutes to remove the supernatant. To each of the precipitates obtained under above Conditions 1 and 2, 50 µl of a TE solution (manufactured by WAKO, 10 mM Tris-HCl, 1 mM EDTA, pH 8) (hereinafter referred to as "lysis reagent") containing 0.005% of yeast RNA (manufactured by SIGMA) in which 0.5 g/ml fused zirconia ZCO-E6 (manufactured by ASTRON, mean diameter of 6 µm) had been suspended was added, and the tube was floated on the washing vessel of the ultrasonic cleaner WT-70ST (manufactured by HONDA ELECTRONICS Co., Ltd., frequency 40 kHz, output power 70 W), and then subjected to an ultrasonic treatment for 5 minutes. It was then centrifuged at 16,000 x g for 3 minutes, and 30 µl of the supernatant was transferred to another tube to obtain a nucleic acid extract.
(1) Nucleic acid extracts obtained as above from a plurality of tuberculosis-negative sputa were mixed to prepare a negative sputum-extracted sample. 4.5 µl of this negative sputum-extracted sample and 0.5 µl of each solution of varying concentrations of standard RNA [a standard RNA containing the base number 243-1843 (the base number was attached in conformity to Z83862 registered in GenBank), which was synthesized by *in vitro* transcription using double stranded DNA containing the sequence of *Mycobacterium* tuberculosis 16S rRNA as the template, and then purified, 1601 bases; the standard RNA was dissolved in a 10 mM Tris-HCl buffer containing 1 mM EDTA and RNase inhibitor; this buffer will be hereinafter be referred to as a "RNA dilution buffer" were mixed, and was subjected to a nucleic acid amplification reaction according to the method described in Japanese Unexamined Patent Publication (Kokai) No. 2004-194583. In the Control (Nega), the negative sputum-extracted sample was replaced and mixed with 4.5 µl of the RNA dilution buffer.
(2) Twenty microliters of a reaction mixture having the following composition was dispensed into a PCR tube (volume 0.5 ml; Gene Amp Thin-Walled Reaction Tube, manufactured by Perkin-Elmer), and the above mixed sample was added thereto.
   Composition of the reaction mixture (each concentration is the concentration in the final volume of 3 0 µl)
   60 mM Tris-HCl buffer (pH 8.6)
   17 mM magnesium chloride
   100 mM potassium chloride
   6 U RNase inhibitor (manufactured by TAKARA BIO INC.)
   1 mM DTT
   0.25 mM each of dATP, dCTP, dGTP, dTTP
   3.6 mM ITP
   3.0 mM each of ATP, CTP, GTP, UTP
   0.16 µM of the first oligonucleotide (MYR-1S-10, SEQ ID NO: 1. The hydroxyl group at the 3'-end was amidated)
   1.0 µM of the second oligonucleotide (MYR-1F-10, SEQ ID NO: 2. A region from "A" at No. 1 to "A" at No. 22 of the 5'-end is the region of T7 promoter, and an ensuing region from "G" at No. 23 to "A" at No. 28 is an enhancer region)
   1.0 µM of the third oligonucleotide (MYR-3RT18, SEQ ID NO: 3)
   25 nM of an oligonucleotide labelled with an intercalating fluorescent dye (YO-MYR-P2-2-S-G, SEQ ID NO: 4. An intercalating dye was labelled in between "A" at No. 7 and "G" at No. 8 of the 5'-end. The hydroxyl group at the 3'-end was modified by glycolic acid) 13% DMSO
   Distilled water for adjusting volume
(3) After keeping the temperature of the above reaction mixture at 43°C for 5 minutes, 5 µl of an enzyme solution that had the following composition and that was previously kept at 43°C for 2 minutes was added:
   Composition of the enzyme solution (each concentration is the concentration in the final volume of 30 µl)
   2.0% sorbitol
   3.6 µg bovine serum albumin
   142 U T7 RNA polymerase (manufactured by TAKARA BIO INC.)
   6.4 U AMV reverse transcriptase (manufactured by TAKARA BIO INC.)
   Distilled water for adjusting volume
(4) Then, while keeping the temperature at 43°C using a fluorescent photometer equipped with a temperature control function that permits direct measurement of the PCR tube, the reaction mixture was measured for a period of time with an excitation wavelength of 470 nm and an emission wavelength of 520 nm.
(5) The result of the rise time (time until a fluorescence increase ratio becomes equal to 1.2 times the value of the sum of the negative mean and three times the standard deviation) of each nucleic acid extract is shown in Fig. 1. These results show that under above condition 1, the inhibiting substances derived from tuberculosis-negative sputa delayed the rise time by 4 minutes or more for 10³ copies of standard RNA, 3 minutes for 10⁴ copies, and 2 minutes or more for 10⁵ copies than that with no extract-addition. On the other hand, under above condition 2, it was improved to 3 minutes for 10³ copies, 1 minute for 10⁴ copies, and 1 minute for 10⁵ copies.

### Example 2

### Pretreatment of tuberculosis-negative sputum and nucleic acid extraction therefrom - 1

To 200 µl of a test sample prepared by subjecting a tuberculosis-negative sputum to a NALC-NaOH treatment (trade name: BBL Mycoprep, Nippon Becton Dickinson Co., Ltd), BCG solutions with 10, 20, 40, 80 and 160 cfu were added and used as test samples.

One ml of the wash reagent was added thereto, allowed to stand on a dry heat block at 70°C for 3 minutes, and then centrifuged at 16,000 x g for 5 minutes to remove the supernatant. To the precipitate obtained, 50 µl of the lysis reagent was added, and the tube was floated on the washing vessel of ultrasonic cleaner VS-D100 (manufactured by HONDA ELECTRONICS Co., Ltd., frequency 24, 31 kHz, output power 110 W), and then subjected to an ultrasonic treatment for 5 minutes. Then it was centrifuged at 16,000 x g for 3 minutes, and 30 µl of the supernatant was transferred to another tube to obtain a nucleic acid extract. Five microliters of it was subjected to a nucleic acid amplification reaction as in Example 1 to measure BCG 16S rRNA.

The result of the rise time (time until a fluorescence increase ratio becomes equal to 1.2 times the value of the sum of the negative mean and three times the standard deviation) of each nucleic acid extract is shown in Fig. 2. These results show that the extraction method of the present invention can detect a BCG in sputum.

### Example 3

It was examined to determine whether the extraction method of the present invention can be applied to a nontuberculous acid-fast bacterium *Mycobacterium avium*.

To 200 µl of test samples prepared by subjecting tuberculosis-negative sputum to a NALC-NaOH treatment (trade name: BBL Mycoprep, Nippon Becton Dickinson Co., Ltd), 10, 20, 40, 80 and 160 cfu of *M. avium* were added and used as test samples.

One ml of the wash reagent was added to the test sample and after mixing, it was allowed to stand on a dry heat block at 70°C for 3 minutes, and then centrifuged at 16,000 x g for 5 minutes to remove the supernatant. To the precipitate obtained, 50 µl of the lysis reagent was added, and the tube was floated on the washing vessel of ultrasonic cleaner VS-D100 (manufactured by HONDA ELECTRONICS Co., Ltd., frequency 24, 31 kHz, output power 110 W), and then subjected to an ultrasonic treatment for 5 minutes. Then it was centrifuged at 16,000 xg for 3 minutes, and 30 µl of the supernatant was transferred to another tube to obtain a nucleic acid extract. Five microliters of it was subjected to a nucleic acid amplification reaction as in Example 1 to measure *M. avium* 16S rRNA. The combinations of the primers and probes used are as follows:
First oligonucleotide (MYR-1S-40, SEQ ID NO: 5)
Second oligonucleotide (MYR-1F-40, SEQ ID NO: 6)
Third oligonucleotide (MYR-3RA16-4, SEQ ID NO: 7)

An oligonucleotide labelled with an intercalating fluorescent dye (YO-MYR-P5-S-G, SEQ ID NO: 8)

The result of the rise time (time until a fluorescence increase ratio becomes equal to 1.2 times the value of the sum of the negative mean and three times the standard deviation) of each nucleic acid extract is shown in Fig. 3. These results show that the extraction method of the present invention can detect each cfu number of *M*. *avium* in sputum.

### Example 4

It was examined to determine whether the extraction method of the present invention can be applied to a nontuberculous acid-fast bacterium *Mycobacterium intracellulare*.

To 200 µl of test samples prepared by subjecting tuberculosis-negative sputum to a NALC-NaOH treatment (trade name: BBL Mycoprep, Nippon Becton Dickinson Co., Ltd), 10, 20, 40, 80 and 160 cfu *M. intracellulare* were added and used as test samples.

One ml of the wash reagent was added to the test sample, a nucleic acid extract was obtained as in Example 3. Five microliters of it was subjected to a nucleic acid amplification reaction as in Example 3 to measure *M. intracellulare* 16S rRNA. The combinations of the primers and probes used are as follows:
First oligonucleotide (MYR-1S-40, SEQ ID NO: 5)
Second oligonucleotide (MYR-1F-40, SEQ ID NO: 6)
Third oligonucleotide (MYR-3RI18, SEQ ID NO: 9)

An oligonucleotide labelled with an intercalating fluorescent dye (YO-MYR-P5-S-G, SEQ ID NO: 8)

The result of the rise time (time until a fluorescence increase ratio becomes equal to 1.2 times the value of the sum of the negative mean and three times the standard deviation) of each nucleic acid extract is shown in Fig. 4. These results show that the extraction method of the present invention can detect each cell number of *M. intracellulare* in sputum.

### Example 5

It was examined to determine whether the extraction method of the present invention can be applied to a nontuberculous acid-fast bacterium *Mycobacterium kansasii.*

To 200 µl of a mucin solution (mucin 2.1 mg/ml, peptone 4.2%, 10 mM Tris-HCl, 1 mM EDTA, pH 8) as a substitute for the NALC-NaOH-treated tuberculosis-negative sputum, 10, 20, 40, 80 and 160 cfu of *M. kansasii* were added and they were used as test samples.

One ml of the wash reagent was added to the test sample, and a nucleic acid extract was obtained as in Example 3. Five microliters of it was subjected to a nucleic acid amplification reaction as in Example 3 to measure *M. kansasii* 16S rRNA. The combinations of the primers and probes used are as follows:
First oligonucleotide (MYR-1S-40, SEQ ID NO: 5)
Second oligonucleotide (MYR-1F-40, SEQ ID NO: 6)
Third oligonucleotide (MYR-3RK16-4, SEQ ID NO: 10)

An oligonucleotide labelled with an intercalating fluorescent dye (YO-MYR-P4-S-G, SEQ ID NO: 11)

The result of the rise time (time until a fluorescence increase ratio becomes equal to 1.2 times the value of the sum of the negative mean and three times the standard deviation) of each nucleic acid extract is shown in Fig. 5. These results show that the extraction method of the present invention can detect each cfu number of *M. kansasii* in sputum.

## Claims

1. A method of extracting biological material-derived nucleic acid from samples containing biological material, said method comprising mixing said biological material with an aqueous solution containing at least a surfactant, subjecting the mixture to a heating treatment and then removing said aqueous solution to separate the biological material, adding a solid powder suspension to said biological material, agitating or sonicating it to disrupt said biological material, and extracting nucleic acid from said biological material.

2. The method of extracting nucleic acid according to claim 1, wherein said surfactant is an anionic surfactant having a steroid backbone or an ampholytic surfactant.

3. The method of extracting nucleic acid according to claim 2, wherein said anion surfactant or ampholytic surfactant is selected from the group consisting of bile acid, cholic acid, deoxycholic acid, 3-[(3-cholamidopropyl)dimethylammonio]propane sulfonic acid (CHAPS) and salts thereof.

4. The method of extracting nucleic acid according to claim 3, wherein the concentration of said surfactant is in the range of 0.1 mM to 50 mM.

5. The method of extracting nucleic acid according to any of claims 1 to 4, wherein the temperature of the heating treatment is 60°C to 90°C.

6. The method of extracting nucleic acid according to any of claims 1 to 5, wherein the above solid powder is amorphous, its long diameter is 32 µm or less, its specific gravity is 3.0 or greater, and its hardness (Hv10) is 600 or greater.

7. The method of extracting nucleic acid according to claim 6, wherein the above amorphous powder is zirconia.

8. A method of extracting biological material-derived nucleic acid from samples containing the
biological material, said method comprising the steps of:
(1) separating the biological material from the above sample, (2) mixing said biological material with an aqueous solution containing, at least, cholic acid at a concentration of 0.1 to 50 mM, (3) subjecting the mixture to a heating treatment at 60°C to 90°C and then removing said aqueous solution to separate the above biological material, (4) adding to the biological material a suspension containing zirconia powder, wherein said powder has an amorphous shape and has a major diameter of 20 µm or less, a specific gravity of 4.5 to 6.5, and a hardness (Hv10) of 800 or greater, (5) disrupting the above biological material by ultrasonication, and (6) obtaining the nucleic acid of interest in the supernatant of the disrupted product.

9. The method of extracting nucleic acid according to claim 8, wherein the above ultrasonication is conducted by treating simultaneously or alternately with ultrasonic waves of two wavelengths.

10. The method of extracting nucleic acid according to any of claims 1 to 9, wherein the above biological material is a virus, microorganism, protozoa, plant or animal tissue.

11. The method of extracting nucleic acid according to claim 10, wherein the above microorganism is one that belongs to the genus *Mycobacterium.*

12. The method of extracting nucleic acid according to any of claims 1 to 11, wherein the sample containing the biological material is an organism-derived sample such as sputum, gastric juice, urine, pus, ascites, pleural effusion, pericardial fluid, blood and tissue, a tissue washing liquid such as a bronchial lavage and an alveolar lavage, culture medium, and an environmental material such as soil, water and air.

## Patentansprüche

1. Verfahren zur Extraktion von von biologischem Material abgeleiteter Nukleinsäure von biologisches Material enthaltenden Proben, wobei das Verfahren ein Mischen des biologischen Materials mit einer mindestens ein Tensid enthaltenden wässrigen Lösung, Unterwerfen der Mischung einer Wärmebehandlung und danach Entfernen der wässrigen Lösung zum Abscheiden des biologischen Materials, Hinzufügen einer Feststoffpulversuspension zum biologischen Material, das zum Aufbrechen des biologischen Materials gerührt oder ultraschallbehandelt wird, und Extraktion der Nukleinsäure aus dem biologischen Material.

2. Verfahren zur Extraktion von Nukleinsäure nach Anspruch 1, wobei das Tensid ein anionisches Tensid mit einem Steroidgerüst oder ein ampholytisches Tensid ist.

3. Verfahren zur Extraktion von Nukleinsäure nach Anspruch 2, wobei das anionische Tensid oder ampholytische Tensid ausgewählt ist der Gruppe bestehend aus Gallensäure, Cholsäure, Desoxycholsäure, (3-[(3-Cholamidopropyl)dimethylammonio]propansulfonsäure) (CHAPS) und Salzen davon.

4. Verfahren zur Extraktion von Nukleinsäure nach Anspruch 3, wobei die Konzentration des Tensids im Bereich von 0,1 mM bis 50 mM beträgt.

5. Verfahren zur Extraktion von Nukleinsäure nach einem der Ansprüche 1 bis 4, wobei die Temperatur der Wärmebehandlung 60 °C bis 90 °C beträgt.

6. Verfahren zur Extraktion von Nukleinsäure nach einem der Ansprüche 1 bis 5, wobei das vorstehend genannte Feststoffpulver amorph ist, sein Längsdurchmesser beträgt 32 µm oder weniger, sein spezifisches Gewicht beträgt 3,0 oder mehr, und seine Härte (Hv10) beträgt 600 oder mehr.

7. Verfahren zur Extraktion von Nukleinsäure nach Anspruch 6, wobei das vorstehend genannte amorphe Pulver Zirconiumdioxid ist.

8. Verfahren zur Extraktion von von biologischem Material abgeleiteter Nukleinsäure von biologisches Material enthaltenden Proben, wobei das Verfahren die folgenden Schritte aufweist:
(1) Abscheiden des biologischen Materials von der vorstehend genannten Probe, (2) Mischen des biologischen Materials mit einer mindestens Cholsäure mit einer Konzentration von 0,1 Mm bis 50 mM enthaltenden wässrigen Lösung, (3) Unterwerfen der Mischung einer Wärmebehandlung bei 60 °C bis 90 °C und anschließend Entfernen der wässrigen Lösung zum Abscheiden des vorstehend genannten biologischen Materials, (4) Hinzufügen zum biologischen Material ein Zircondioxid enthaltendes Pulver, wobei das Pulver amorphe Form und einen größten Durchmesser von 20 µm oder weniger, ein spezifisches Gewicht von 4,5 bis 6,5 und eine Härte (Hv10) von 800 oder mehr aufweist, (5) Aufbrechen des vorstehend genannten biologische Material durch Ultraschallbehandlung und (6) Gewinnen der Nukleinsäure von Interesse im Überstand des aufgebrochenen Produkts.

9. Verfahren zur Extraktion von Nukleinsäure nach Anspruch, 8, wobei die vorstehend genannte Ultraschallbehandlung durch gleichzeitiges oder abwechselndes Beaufschlagen mit Ultraschallwellen von zwei Wellenlängen durchgeführt wird.

10. Verfahren zur Extraktion von Nukleinsäure nach einem der Ansprüche 1 bis 9, wobei das vorstehend genannte biologische Material ein Virus, Mikroorganismus, Protozoon, pflanzliches oder tierisches Gewebe ist.

11. Verfahren zur Extraktion von Nukleinsäure nach Anspruch, 10, wobei der vorstehend genannte Mikroorganismus ein der Gattung *Mycobacterium* zugehöriger ist-

12. Verfahren zur Extraktion von Nukleinsäure nach einem der Ansprüche 1 bis 11, wobei die das biologische Material enthaltende Probe eine von einem Organismus abgeleitete Probe, wie Sputum, Magensaft, Urin, Eiter, Aszites, Pleuraerguss, Perikardflüssigkeit, Blut und Gewebe, eine Gewebewaschflüssigkeit wie Bronchiallavage und eine alveoläre Lavage, Nährmedium und ein Umweltmaterial wie Erde, Wasser und Luft ist.

## Revendications

1. Procédé d'extraction d'acide nucléique dérivé de matériel biologique à partir d'échantillons contenant un matériel biologique, ledit procédé comprenant le fait de mélanger ledit matériel biologique avec une solution aqueuse contenant au moins un tensioactif, de soumettre le mélange à un traitement thermique et puis de retirer ladite solution aqueuse pour séparer le matériel biologique, d'ajouter une suspension de poudre solide audit matériel biologique, d'agiter ou d'effectuer une sonication de celle-ci pour rompre ledit matériel biologique, et d'extraire l'acide nucléique à partir dudit matériel biologique.

2. Procédé d'extraction d'acide nucléique selon la revendication 1, dans lequel ledit tensioactif est un tensioactif anionique ayant un squelette stéroïde ou un tensioactif ampholyte.

3. Procédé d'extraction d'acide nucléique selon la revendication 2, dans lequel ledit tensioactif anionique ou ledit tensioactif ampholyte est choisi dans le groupe constitué d'acide biliaire, d'acide cholique, d'acide désoxycholique, d'acide 3-[(3-cholamidopropyl)diméthylammonio]propane sulfonique (CHAPS) et de sels de ceux-ci.

4. Procédé d'extraction d'acide nucléique selon la revendication 3, dans lequel la concentration dudit tensioactif se trouve dans la plage allant de 0,1 mM à 50 mM.

5. Procédé d'extraction d'acide nucléique selon l'une des revendications 1 à 4, dans lequel la température du traitement thermique est comprise entre 60°C et 90°C.

6. Procédé d'extraction d'acide nucléique selon l'une des revendications 1 à 5, dans lequel la poudre solide ci-dessus est amorphe, son diamètre long est inférieur ou égal à 32 µm, sa densité est supérieure ou égale à 3,0, et sa dureté (Hv10) est supérieure ou égale à 600.

7. Procédé d'extraction d'acide nucléique selon la revendication 6, dans lequel la poudre amorphe ci-dessus est de la zircone.

8. Procédé d'extraction d'acide nucléique dérivé de matériel biologique à partir d'échantillons contenant le matériel biologique, ledit procédé comprenant les étapes qui consistent : (1) à séparer le matériel biologique de l'échantillon ci-dessus, (2) à mélanger ledit matériel biologique avec une solution aqueuse contenant, au moins, de l'acide cholique à une concentration allant de 0,1 à 50 mM, (3) à soumettre le mélange à un traitement thermique à une température allant de 60°C à 90°C et puis à retirer ladite solution aqueuse pour séparer le matériel biologique ci-dessus, (4) à ajouter au matériel biologique une suspension contenant une poudre de zircone, où ladite poudre présente une forme amorphe et a un diamètre majeur inférieur ou égal à 20 µm, une densité allant de 4,5 à 6,5, et une dureté (Hv10) supérieure ou égale à 800, (5) à rompre le matériel biologique ci-dessus par ultrasonication, et (6) à obtenir de l'acide nucléique d'intérêt dans le surnageant du produit rompu.

9. Procédé d'extraction d'acide nucléique selon la revendication 8, dans lequel l'ultrasonication ci-dessus est réalisée par traitement de manière simultanée ou alternée avec des ondes ultrasonores de deux longueurs d'onde.

10. Procédé d'extraction d'acide nucléique selon l'une des revendications 1 à 9, dans lequel le matériel biologique ci-dessus est un virus, un micro-organisme, un protozoaire, un tissu végétal ou animal.

11. Procédé d'extraction d'acide nucléique selon la revendication 10, dans lequel le micro-organisme ci-dessus est celui qui appartient au genre *Mycobacterium.*

12. Procédé d'extraction d'acide nucléique selon l'une des revendications 1 à 11, dans lequel l'échantillon contenant le matériel biologique est un échantillon dérivé d'organisme tel que le crachat, le suc gastrique, l'urine, le pus, les ascites, l'épanchement pleural, un fluide péricardique, du sang et un tissu, un liquide de lavage de tissu tel qu'un milieu de lavage bronchique et un milieu de lavage alvéolaire, un milieu de culture, et un matériau de l'environnement tel que le sol, l'eau et l'air.
